# EUROPÄISCHE PATENTSCHRIFT

(11) **EP 1 771 576 B1**
(45) Veröffentlichungstag und Bekanntmachung des Hinweises auf die Patenterteilung: **16.10.2013**
(21) Anmeldenummer: 05762739.0
(22) Anmeldetag: 07.07.2005
(51) Int. Cl.: C12Q 1/68, G01N 33/50

(54) **WIRKSTOFFE ZUR STEIGERUNG DER PATHOGENABWEHR IN PFLANZEN UND METHODEN ZU IHRER AUFFINDUNG**
ACTIVE SUBSTANCES FOR INCREASING PATHOGENIC DEFENCE IN PLANTS AND METHODS FOR THE DETECTION THEREOF
PRINCIPES ACTIFS PERMETTANT D'AUGMENTER LA DEFENSE DES PLANTES CONTRE LES PATHOGENES ET LEURS PROCEDES DE DETECTION

(30) Priorität: 20.07.2004 DE 102004035137
(43) Veröffentlichungstag der Anmeldung: 11.04.2007
(73) Patentinhaber: Bayer CropScience AG, 40789 Monheim (DE)
(72) Erfinder: BARTSCH, Klaus, 61462 Königstein (DE); SCHULZ, Arno, 65817 Eppstein (DE)
(86) Internationale Anmeldenummer: PCT/EP2005/007344
(87) Internationale Veröffentlichungsnummer: WO 2006/007981

(56) Entgegenhaltungen:
- EP-A- 1 013 767
- WO-A-98/00023
- WO-A2-02/060255
- FEUSSNER I ET AL: "Induction of a new lipoxygenase form of cucumber leaves by salicylic acid or 2,6-dichloroisonicotinic acid" BOTANICA ACTA, STUTTGART, DE, Bd. 110, 1997, Seiten 101-108, XP002105415 ISSN: 0932-8629
- GÖRLACH J ET AL: "Benzothiadiazole, a novel class of inducers of systemic acquired resistance, activates gene expression and disease resistance in wheat" PLANT CELL, AMERICAN SOCIETY OF PLANT PHYSIOLOGISTS, ROCKVILLE, MD, US, Bd. 8, Nr. 4, April 1996 (1996-04), Seiten 629-643, XP002105413 ISSN: 1040-4651
- SCHENK P M ET AL: "Coordinated plant defense responses in Arabidopsis revealed by microarray analysis" PROCEEDINGS OF THE NATIONAL ACADEMY OF SCIENCES OF USA, NATIONAL ACADEMY OF SCIENCE, WASHINGTON, DC, US, Bd. 97, Nr. 21, 10. Oktober 2000 (2000-10-10), Seiten 11655-11660, XP002153163 ISSN: 0027-8424
- ZHU-SALZMAN KEYAN ET AL: "Transcriptional regulation of sorghum defense determinants against a phloem-feeding aphid." PLANT PHYSIOLOGY. JAN 2004, Bd. 134, Nr. 1, Januar 2004 (2004-01), Seiten 420-431, XP002348850 ISSN: 0032-0889
- REYMOND P ET AL: "Differential gene expression in response to mechanical wounding and insect feeding in Arabidopsis", THE PLANT CELL, AMERICAN SOCIETY OF PLANT BIOLOGISTS, US, vol. 12, no. 5, 1 May 2000 (2000-05-01), pages 707-719, XP002216347, ISSN: 1040-4651, DOI: 10.1105/TPC.12.5.707
- WASTERNACK CLAUS ET AL: "Jasmonic acid: Biosynthesis, signal transduction, gene expression", FETT, vol. 100, no. 4-5, May 1998 (1998-05), pages 139-146, ISSN: 0931-5985

## Beschreibung

Beschrieben werden ein Verfahren zum Auffinden von die Pathogenabwehr von Pflanzen induzierenden Verbindungen, wobei die Steigerung der Expression einzelner oder mehrerer pflanzenendogener Gene aus der Gruppe der Jasmonsäurebiosynthese, pflanzlicher Proteinase-Inhibitoren, pflanzlicher Xylanase-Inibitoren, pflanzlicher PR-Proteine (pathogen-related proteins) und pflanzlicher Chitinasen als Indiz für die Induktion betrachtet wird, sowie die Verwendung dieser Verbindungen allein oder in Kombination mit bereits bekannten gegenüber Phytotathogenen spezifisch und direkt wirksamen Verbindungen, wobei die Applikation entweder gleichzeitig oder zeitlich versetzt erfolgen kann.

Die Verfahren und Verwendungen der Erfindung sind in den angefügten Ansprüchen offenbart.

Es ist bekannt, daß Pflanzen auf natürliche Stressbedingungen, wie beispielsweise Kälte, Hitze, Trockenheit, Verwundung, Pathogenbefall (Viren, Bakterien, Pilze, Insekten) etc. aber auch auf Herbizide mit spezifischen oder unspezifischen Abwehrmechanismen reagieren [Pflanzenbiochemie, S. 393-462 , Spektrum Akademischer Verlag, Heidelberg, Berlin, Oxford, Hans W. Heldt, 1996.; Biochemistry and Molecular Biology of Plants, S. 1102-1203, American Society of Plant Physiologists, Rockville, Maryland, eds. Buchanan, Gruissem, Jones, 2000]. Dabei dienen z.B. durch Verwundung entstandene Zellwandbestandteile oder spezifische vom Pathogen stammende Signalsubstanzen als Induktoren pflanzlicher Signaltransduktionsketten, die am Ende zur Bildung von gegen den Stressfaktor gerichteten Abwehrmolekülen führen. Hierbei kann es sich beispielsweise um (a) niedermolekulare Substanzen, wie z.B. Phytoalexine, (b) nicht-enzymatische Proteine, wie z.B. "Pathogen-related proteins" (PR-Proteine), (c) enzymatische Proteine, wie beispielsweise Chitinasen, Glucanasen, oder (d) um spezifische Inhibitoren essentieller Proteine, wie beispielsweise um Protease-Inhibitoren, Xylanase-Inhibitoren, handeln, welche das Pathogen direkt angreifen oder seine Proliferation behindern (Dangl and Jones, 2001, Nature 411: 826-833; Kessler and Baldwin, 2003, Annual Review of Plant Biology, 53: 299-328).

Ein zusätzlicher Abwehrmechanismus ist die sogenannte hypersensitive Reaktion (HR), die über oxidativen Stress vermittelt wird und zum Absterben von Pflanzengewebe im Bereich eines Infektionsherdes führt, wodurch eine Ausbreitung von Pflanzenpathogenen, die auf lebende Zellen angewiesen sind, verhindert wird [Pennazio, 1995, New Microbiol. 18, S. 229-240].

Im weiteren Verlauf einer Infektion werden durch pflanzeneigene Botenstoffe Signale in nicht befallene Gewebe weitergegeben, die auch dort zur Auslösung von Abwehrreaktionen führen und die Entstehung von Sekundärinfektionen behindern (Systemic acquired resistance, SAR) [Ryals et al., 1996, The Plant Cell 8: 1809-1819].

Eine Reihe von pflanzenendogenen Signalstoffen, die in die Stresstoleranz bzw. die Pathogenabwehr involviert sind, sind bereits bekannt. Zu nennen sind hier beispielsweise Salicylsäure, Benzoesäure, Jasmonsäure oder Ethylen [Biochemistry and Molecular Biology of Plants, S. 850-929, American Society of Plant Physiologists, Rockville, Maryland, eds. Buchanan, Gruissem, Jones, 2000]. Einige dieser Substanzen oder deren stabile synthetische Derivate und abgeleitete Strukturen sind auch bei externer Applikation auf Pflanzen oder Saatgutbeizung wirksam und aktivieren Abwehrreaktionen, die eine erhöhte Stress- bzw. Pathogentoleranz der Pflanze zur Folge haben [Sembdner, and Parthier, 1993, Ann. Rev. Plant Physiol. Plant Mol. Biol. 44: 569-589]. Die Salicylat-vermittelte Abwehr richtet sich besonders gegen phytopathogene Pilze, Bakterien und Viren [Ryals et al., 1996, The Plant Cell 8: 1809-1819].

Ein bekanntes synthetisches Produkt, das eine der Salicylsäure vergleichbare Funktion übernimmt und eine Schutzwirkung gegen phytopathogene Pilze, Bakterien und Viren vermitteln kann, ist Benzothiadiazol (Handelsname Bion^{®}) [Achuo et al., 2004, Plant Pathology 53 (1): 65-72].

Andere Verbindungen, die in die Gruppe der Oxylipine gehören, wie z.B. Jasmonsäure, und die durch sie ausgelösten Schutzmechanismen sind besonders gegen Schadinsekten wirksam [Walling, 2000, J Plant Growth Regul. 19, 195-216]

WO 98/00023 offenbart ein Screeningverfahren zum Auffinden von Substanzen, die über Aktivierung der Jasmonsäurebiosynthese die Pflanzen vor Pathogenen schützt.

Somit ist bekannt, daß Pflanzen über mehrere endogene Reaktionsmechanismen verfügen, die eine wirksame Abwehr gegenüber verschiedensten Schadorganismen und/oder natürlichen abiotischen Stress bewirken können. Eine Voraussage darüber, welche Abwehrreaktionen gezielt durch Anwendung von Wirkstoffen hervorgerufen werden können, war bisher jedoch nicht bekannt.

Es besteht somit ein Bedarf nach einer Methode zum gezielten Auffinden molekularer Aktivatoren pflanzenendogener Abwehrmechanismen gegenüber Schadorganismen und/oder natürlichen abiotischen Stress (wie beispielsweise Hitze, Kälte, Trockenheit, Salinität, sowie Säuren-/und Basenbelastung) wodurch neuartige Wirkstoffe aufgefunden, neue Eigenschaften bekannter, aber andersartig wirkender Wirkstoffe identifiziert, oder aber bereits bekannte Moleküle oder Leitstrukturen auf die Verwendung als Induktoren der pflanzenendogenen Abwehrmechanismen optimiert werden können.

### Definitionen nachfolgend verwendeter Begriffe

Der Begriff "Blast-Analysen" (Blast = Basic Local Alignment Search Tool)", wie hier verwendet, beschreibt die Verwendung geeigneter Computer-Programme zur Klassifikation und zum Auffinden potentiell homologer Sequenzen (Altschul et al., J. Mol. Biol. 1990, 215: 403-410), wobei ein Vergleich (Alignment) zwischen einer Suchsequenz (query sequence) und allen Sequenzen einer oder mehrerer Datenbanken unter Vorgabe einer gewünschten Übereinstimmung in Form eines "Signifkanz-Kriteriums" (scoring function), erfolgt (R. Rauhut, Bioinformatik, S 38-107, Verlag Wiley-VCH Verlag GmbH, Weinheim, 2001).

Der Begriff "cDNA" (complementary DNA), wie hier verwendet, beschreibt einen DNA-Einzelstrang, der zu einer RNA komplementär, und der durch eine enzymatische reverse Transkription *in vitro* synthetisiert wird. Die cDNA kann entweder der gesamten RNA-Länge entsprechen, oder aber nur eine Teilsequenz der als Matrix dienenden RNA darstellen.

Der Begriff "Cluster-Analyse", wie hier verwendet, bedeutet die Zusammenfassung der ermittelten Einzeldaten mittels eines hierfür entwickelten Computerprogramms, wobei Gruppen von Genen, die für Proteine mit ähnlicher Funktion kodieren, oder aber Gene mit ähnlichem Expressionsmuster zusammenfassend dargestellt werden. Hierdurch wird eine hierarchische Minimierung des komplexen Datenmusters erreicht, die in Form eines Dendrograms dargestellt werden kann. Die Cluster-Analyse ermöglicht die klassifizierende Bewertung der erhaltenen Datensätze, die deutlich über die bloße Akkumulierung beziehungsloser Daten hinausgeht.

Unter den Begriffen "DNA-Chip", "DNA-Array" und "DNA-Microarray", die hier synonym verwendet werden, wird ein Träger bezeichnet dessen Grundmaterial beispielsweise aus Glas oder Nylon besteht, auf dessen Grundmaterial DNA-Fragmente fixiert sind, wobei die Aufbringung der DNA beispielsweise durch (a) ein photolithographisches Verfahren (DNA wird direkt auf dem Arrayträger synthetisiert), (b) ein Microspotting-Verfahren (extern synthetisierte Oligonukleotide oder PCR-Produkte werden auf den Träger appliziert und kovalent gebunden), oder (c) durch eine Microspraying-Verfahren (extern synthetisierte Oligonukleotide oder PCR-Produkte werden mit einem Tintenstrahldrucker berührungsfrei auf den Träger gesprüht) erfolgen kann (R. Rauhut, Bioinformatik, S 197-199, Verlag Wiley-VCH Verlag GmbH, Weinheim, 2001). Ein DNA-Chip, der genomische Sequenzen eines Organismus representiert, wird als "genomischer DNA-Chip" bezeichnet. Die Auswertung der mit Hilfe dieser DNA-Chips" erhaltenen Meßwerte wird als "DNA-Chip-Analyse" bezeichnet.

Der Begriff "DNA-Chip-Hybridisierung", wie hier verwendet, bedeutet die Paarung zweier einzelstränger, komplementärer Nukleinsäuremoleküle, wobei eines der basenpaarenden Molekülpartner als DNA (Desoxribonukleinsäure) auf dem DNA-Chip in bevorzugt kovalent gebundener Form lokalisiert ist, während der andere in Form der RNA (Ribonukleinsäure) oder der hierzu korrespondierenden cDNA (komplementäre DNA) in Lösung vorliegt. Die Hybridisierung der gebundenen und nicht gebundenen Nukleinsäuren erfolgt auf dem DNA-Chip in wäßriger Pufferlösung, gegebenenfalls unter zusätzlich denaturierenden Bedingungen, wie beispielsweise in Gegenwart von Dimethylsulfoxid, bei Temperaturen von 30-60°C, bevorzugt 40-50°C, besonders bevorzugt bei 45°C für 10-20 Stunden, bevorzugt für 14-18 Stunden, besonders bevorzugt für 16 Stunden unter ständiger Bewegung. Die Hygbridisierungsbedingungen können konstant beispielsweise in einem Hybridisierungsofen realisiert werden. Standardmäßig werden in einem solchen Hybridisierungsofen Bewegungen von 60 rpm (rounds per minute, Umdrehungen pro Minute) realisiert.

Die mit dem Begriff "EST-Sequenz" (Expressed Sequence Tag) bezeichnete Nukleinsäuresequenz, wie hier verwendet, bedeutet eine kurze Sequenz von 200-500 Basen oder Basenpaaren.

Die synonym gebrauchten Begriffe "Expressionsmuster", "Induktionsmuster" bzw. "Expressionsprofil", wie hier verwendet, beschreiben die zeitlich differenzierte und/oder gewebespezifische Expression der pflanzlichen mRNA, wobei das Muster direkt durch die erzeugte Intensität des Hybridisierungssignals der aus der Pflanze erhaltenen RNA oder deren korrespondierender cDNA mit Hilfe der DNA-Chip-Technologie erhalten wird. Die gemessenen "Expressionswerte" ergeben sich durch direkte Verrechnung mit den korrespondierenden Signalen, die durch Verwendung eines synonymen Chips unter Hybridisierung mit einer nicht behandelten Kontrollpflanze erhalten werden.

Der Begriff "Expressionszustand" der durch das vorgenommene "Gene Expression Profiling" erhalten wird, wie hier verwendet, beschreibt die gesamte erfaßte Transkriptionsaktivität zellulärer Gene, die mit Hilfe eines DNA-Chips gemessen wird.

Der Begriff "Gesamt-RNA", wie hier verwendet, beschreibt die aufgrund des angewendeten Aufschlußverfahrens mögliche Repräsentanz verschiedener pflanzenendogener RNA-Gruppen, die in einer Pflanzenzelle vorliegen können, wie beispielsweise, cytoplasmatische rRNA (ribosomale RNA), cytoplasmatische tRNA (transfer RNA), cytoplasmatische mRNA (messenger RNA), sowie deren jeweilige nucleäre Vorläufer, ctRNA (chloroplastidäre RNA) und mtRNA (mitochondriale RNA), sie umfaßt aber auch RNA-Moleküle, die von exogenen Organismen stammen können, wie beispielsweise von Viren, oder parasitierenden Bakterien und Pilzen.

Der Begriff "Nutzpflanzen", wie hier verwendet, bezeichnet Kulturpflanzen, die als Pflanzen für die Gewinnung von Nahrungsmitteln, Futtermitteln oder für technische Zwecke eingesetzt werden.

Der Begriff "Safener", wie hier verwendet, bezeichnet eine chemische Verbindung, die nicht pflanzenendogenen Ursprungs ist, und die die phytotoxischen Eigenschaften eines Pestizids gegenüber Nutzpflanzen aufhebt oder verringert, ohne daß die pestizide Wirkung gegenüber Schadorganismen, wie beispielsweise Unkräutern, Bakterien, Viren und Pilzen wesentlich vermindert wird.

Gegenstand der vorliegenden Offenbarung ist ein Verfahren zum Auffinden einer die Pathogenabwehr von Pflanzen induzierenden Verbindung, wobei die Steigerung der Transkription bzw. Expression einzelner oder mehrerer, pflanzenendogener Gene aus der Gruppe der Jasmonsäurebiosynthese, pflanzlicher Proteinase-Inhibitoren, pflanzlicher Xylanase-Inibitoren, pflanzlicher PR-Proteine (pathogen-related proteins) und pflanzlicher Chitinasen als Indiz für die Induktion bewertet wird.

Gegenstand der vorliegenden Offenbarung ist im besonderen ein Verfahren zum Auffinden von Verbindungen, die die Transkription der für pflanzenendogene Enzyme der Pathogenabwehr kodierenden Gene induzieren, dadurch gekennzeichnet, daß:
a) Testpflanzen mit einer geeigneten Menge der Testsubstanz oder den Testsubstanzen in Kontakt gebracht werden,
b) Kontrollpflanzen, unter ansonsten gleichen Bedingungen wie Testpflanzen unter a), nicht mit der oder den Testsubstanzen in Kontakt gebracht werden,
c) RNA aus den Test- und Kontrollpflanzen extrahiert wird,
d) die RNA entweder direkt radioaktiv oder nicht radioaktiv markiert wird, oder aber die RNA unter gleichzeitiger enzymatischer Umschreibung in die korrespondierende cDNA radioaktiv oder nicht-radioaktiv markiert wird, oder aber die erhaltene, nicht markierte cDNA enzymatisch in eine korrespondierende radioaktive oder nicht-radioaktive markierte cRNA umgeschrieben wird,
e) ein pflanzliche DNA-Sequenzen enthaltender DNA-Array mit den gemäß Schritt d) erhaltenen Substanzen hybridisiert wird,
f) Expressionsprofile der Gene für die Expression der Jasmonsäurebiosynthese, pflanzlicher Proteinase-Inhibitoren, pflanzlicher Xylanase-Inhibitoren, pflanzlicher PR-Proteine (pathogen-related proteins) und/oder pflanzlicher Chitinasen vergleichend für die gemäß a) und b) getesteten Pflanzen erstellt werden,
g) eine Quantifizierung der gemäß f) gemessenen Expressionsunterschiede erfolgt,
h) eine abschließende Systematisierung der gemäß g) zugeordneten Expressionsprofile durch Cluster-Analyse erfolgt.

Im Fall des zuvor genannten Schritts d) ist die enzymatische Umschreibung der erhaltenen cDNA in eine cRNA als bevorzugter Verfahrensschritt anzusehen, da hierdurch eine nochmalige Amplifikation der Hybridisierungsprobe erreicht werden kann. Ebenso bevorzugt ist die Markierung mittels nicht radioaktiver Nukleotide, besonders bevorzugt die Markierung mittels biotinyliertem UTP und/oder CTP, wobei der Nachweis im Anschluß an die erfolgte Hybridisierungsreaktion durch Bindung von Streptavidin-Phycoerythrin als Fluorophor and die biotinylierte cRNA erfolgt. Ein Nachweis der spezifischen Phycoerythrin-Fluoreszenz, die als Grundlage für die Quantifizierung der gemessenen Expressionsunterschiede dient, erfolgt im Anschluß an die Hybridisierung mit Hilfe eines Laser-Scanners.

Bevorzugter Gegenstand der vorliegenden Offenbarung ist ein Verfahren unter Einhalten der zuvor genannten Verfahrensabläufe a) - h), wobei
(i) das Expressionsprofil eines Gens des Lipase-like Proteins, der 12-Oxophytodienoate Reductase (EC 1.3.1.42), der Allene-oxide Cyclase, der 12-oxophytodienoic acid Reductase, und/oder
(ii) das Expressionsprofil eines Gens eines pflanzlichen Proteinase-Inhibitors, der signifikante Homologien zum Proteinase-Inhibitor mit dem Eintrag in der PIR-Proteindatenbank S71555 aufweist, und/oder
(iii) das Expressionsprofil eines Gens eines pflanzlichen Xylanase Inhibitor Proteins, und/ oder
(iv) das Expressionsprofil eines Gens der pflanzlichen pathogen-induzierten Peroxidase (EC 1.11.1.7), einem Protein, das signifikante Homologien zum Pathogen Related (PR) protein der PIR-Proteindatenbank mit der Nummer T06168 aus Gerste aufweist, und/oder
(v) das Expressionsprofil eines Gens der pflanzlichen Chitinase
gegenüber einer unbehandelten Kontrollpflanze beispielsweise um den Faktor 2 oder mehr, vorzugsweise um den Faktor 2-100, bevorzugt 2-20, besonders bevorzugt 2-10, ganz besonders bevorzugt 2-5, erhöht ist, wobei die Steigerung der geänderten Expressionsprofile der einzelnen Gene unabhängig voneinander in den unterschiedlichen zuvor benannten Größenbereichen liegen kann.

Gegenstand der vorliegenden Offenbarung ist weiter die Verwendung bestimmter DNA-Microarrays, die auf der Basis genetischer Informationen aus Pflanzen, bevorzugt genetischer Information aus Nutzpflanzen, besonders bevorzugt aus Nutzpflanzen wie beispielsweise aus Gerste, Mais, Weizen, Reis, Hafer, Raps, Zuckerrübe zur Auffindung von geänderten Genexpressionsmustern benutzt werden. Dabei werden die relativen Veränderungen der Genmuster für Gene der Jasmonsäurebiosynthese, pflanzlicher Proteinase-Inhibitoren, pflanzlicher Xylanase-Inibitoren, pflanzlicher PR-Proteine (pathogen-related proteins) und/oder pflanzlicher Chitinasen in mit den zu testenden Verbindungen behandelten Pflanzen im Vergleich zu unbehandelten Kontrollpflanzen betrachtet.

Weiterer Gegenstand der vorliegenden Offenbarung ist auch die Verwendung der Verbindungen, die mit dem zuvor genannten Verfahren positiv, d.h. expressionssteigernd hinsichtlich ihrer induktiven Wirkung auf Gene der Jasmonsäurebiosynthese, pflanzlicher Proteinase-Inhibitoren, pflanzlicher PR-Proteine (pathogen-related proteins) und/oder pflanzlicher Chitinasen , identifiziert wurden, als Wirkstoffe zur Steigerung der Stresstoleranz und/oder Pathogenabwehr bei Nutzpflanzen.

Gegenstand der vorliegenden Offenbarung ist daher auch die Verwendung von Verbindungen, die in Pflanzen, direkt oder indirekt, wie beispielsweise durch eine Signaltransduktionskette, zur Steigerung der Abwehrkraft gegenüber phytopathogenen Organismen, wie beispielsweise Insekten, Pilze, Bakterien oder Viren beitragen, wobei mindestens ein Gen, bevorzugt mehrere Gene die für Proteine aus der Gruppe der Proteine der Jasmonsäurebiosynthese, pflanzlicher Proteinase-Inhibitoren, pflanzlicher Xylanase-Inibitoren, pflanzlicher PR-Proteine (pathogen-related proteins) und/oder pflanzlicher Chitinasen, ein gesteigertes Expressionsprofil aufweist.

Bevorzugt sind hierbei solche Verbindungen, die in ihrer Verwendung als sogenannte Safener bereits im Pflanzenschutz bekannt sind, wie beispielsweise, Mefenpyr-dimethyl, Mefenpyr-diethyl, Mefenpyr-Analoga, Isoxadifen-ethyl, Cloquintocet-mexyl, Cloquintocet-Derivate, sowie Pyridincarboxamid.

In der Verwendung der Erfindung ist die Verbindung Isoxadifen-ethyl.

Durch Applikation der zuvor genannten Verbindungen können Nutzpflanzen effektiv gegen phytopathogene Organismen (Insekten, Pilze, Bakterien, Viren) geschützt werden, was sich z.B. auch in höheren Erträgen niederschlägt. Vorteilhaft gegenüber Wirkstoffen, welche direkt gegen diese Organismen gerichtet sind, ist, daß Nützlinge geschont werden, da sie die entsprechenden Abwehrreaktionen nicht auslösen können.

Gegenstand der vorliegenden Offenbarung ist daher auch ein Verfahren zum Schutz von Nutzpflanzen in Nutzpflanzenkulturen gegen phytopathogene Organismen, besonders gegenüber Insekten, Pilzen, Bakterien und Viren durch Applikation der mit Hilfe des DNA-Arrays unter Betrachtung der Expressionsprofile der Gene der Jasmonsäurebiosynthese, pflanzlicher Proteinase-Inhibitoren, pflanzlicher Xylanase-Inibitoren, pflanzlicher PR-Proteine (pathogen-related proteins) und/oder pflanzlicher Chitinasen identifizierten Verbindungen. Ganz besonders bevorzugt ist der Schutz gegenüber phytopathogenen Organismen und hierbei im besonderen gegenüber Insekten, ganz besonders gegenüber saugenden Insekten.

Die genannten, durch die mit Hilfe der DNA-Arrays erfaßten Verbindungen, wie auch beispielsweise durch bereits als Safener bekannte Verbindungen, ausgelösten Mechanismen führen auch zu Synergieeffekten zwischen diesen Verbindungen und weiteren spezifisch und direkt gegen Phytopathogene wirksame Verbindungen, wie beispielsweise Insektizide und Fungizide, besonders in gemeinsamer oder zeitlich versetzter Applikation mit Insektiziden und Fungiziden, vor allem mit Insektiziden, wie z.B. Verbindungen aus der Gruppe der Ketoenole [z.B. in EP 528156; EP 596298] oder Agonisten bzw. Antagonisten des nicotinergen Acetylcholinrezeptors. Letztere Verbindungen werden zum Teil unter dem Begriff Nitromehtylene oder Nitroimine und mit diesen verwandte Verbindungen zusammengefasste (z.B. in EP 464830).

Gegenstand der vorliegenden Offenbarung ist daher auch der Verwendung von den durch das Screening mittels eines DNA-Arrays unter Betrachtung der Expressionsprofile der Gene der Jasmonsäurebiosynthese, pflanzlicher Proteinase-Inhibitoren, pflanzlicher Xylanase-Inibitoren, pflanzlicher PR-Proteine (pathogen-related proteins) und/oder pflanzlicher Chitinasen identifizierten Verbindungen in Kombination mit bereits bekannten, gegenüber Phytotathogenen spezifisch und direkt wirksamen Verbindungen, wie beispielsweise die Kombination von Mefenpyr-Derivaten (wie beispielsweise die im "The Pesticide Manual, 13th Edition, 2003" unter Nr. 506 aufgeführte Verbindung) mit direkt auf den Schadorganismus wirkenden Insektiziden, besonders bevorzugt mit einem Insektizid aus der Gruppe der Ketoenole oder der Gruppe der Nitromethylene/Nitroimine, oder die Verwendung von Isoxadifen-Derivaten (wie beispielsweise die im "The Pesticide Manual, 13th Edition, 2003" unter Nr. 478 aufgeführte Verbindung) mit direkt auf den Schadorganismus wirkenden Insektiziden, wobei die Applikation entweder gleichzeitig oder zeitlich versetzt erfolgen kann. Bei nicht gleichzeitig erfolgender Applikation kann das direkt auf den Schadorganismus wirkende Insektizid entweder vor oder nach Applikation der mittels des hier beschriebenen DNA-Arrays identifizierten Verbindung appliziert werden, wobei eine subsequentielle Applikation des direkt auf den Schadorganismus wirkenden Insektizids bevorzugt ist.

Die folgenden Beispiele beschreiben die Erfindung im einzelnen.

### Beispiel 1

Nachweis der Wirkung von Safenern auf Abwehrmechanismen in Pflanzen durch Gene Expression Profiling (GEP):
Gerstepflanzen (Varietät Baronesse) wurden in Töpfen mit Erde (Durchmesser: 10 cm) für 10 Tage in einer Klimakammer unter definierten Licht-, Feuchtigkeits- und Temperaturbedingungen (Weißlicht, 70 % Luftfeuchtigkeit, 24°C) angezogen. Zum Zeitpunkt der Spritzapplikation hatten die Keimlinge eine Größe von ca. 15 cm. Als Testsubstanzen wurden in diesem Beispiel sich strukturell deutlich unterscheidende Moleküle ausgewählt, für die eine starke Safener-Wirkung belegt ist, sowie andere Moleküle mit aus der Literatur bekannter Wirkung auf die pflanzliche Stress- und Pathogentoleranz (Tabelle 1). Die Substanzen wurden als Stammlösungen in DMSO (Dimethylsulfoxid) mit c = 10 mg/ml angesetzt. Daraus wurden Verdünnungen in Wasser mit 0,2 % Agrotin (enthält Polyvinylalkohiol, Silikone, Polysacchararide und pH-Regulatoren, Hersteller Bayer CropScience AG, Monheim, Deutschland), als Netzmittel entsprechend den in der Tabelle angegebenen Aufwandmengen hergestellt. Die Flüssigkeitsmenge der Spritzapplikation betrug 800 µl pro Topf entsprechend 800 l/ha. Auf 800 µl Spritzbrühe wurden noch 16 µl EC-Vormischung : Diacetonalkohol = 1:6 als Formulierungshilfsstoffe hinzugefügt. Die in der Tabelle 1 aufgeführten Substanzen wurden mittels einer pneumatischen Spritzpistole auf die Blätter aufgebracht. Es wurden jeweils 2 Substanzdosierungen gespritzt und alle Versuche in Replikaten ausgeführt (2 Töpfe pro Substanz). Als Kontrollen wurden Spritzungen mit Blindformulierung ohne Wirkstoff durchgeführt. Nach 6 h wurden die Blätter geerntet in Flüssig-Stickstoff schockgefroren und bis zur Aufarbeitung bei -80°C gelagert. Die Herstellung der markierten RNA-Sonden für die DNA-Chip-Hybridisierung erfolgte nach den Protokollen (Expression Analysis, Technical Manual) der Firma Affymetrix (Affymetrix Inc., 3380 Central Expressway, Santa Clara, CA, USA). Aus je 500 mg der geernteten Blätter wurde zunächst Gesamt-RNA isoliert. Je 10 µg Gesamt-RNA wurden für die Erst- und Zweitstrang cDNA-Synthese verwendet. Die cDNA wurde mit T7-Polymerase amplifiziert und dabei gleichzeitig mit Biotin-UTP markiert. Je 20 µg dieser biotinylierten cDNA wurden für die Hybridiserung des Gerste Genom-Arrays (Gene Chip Barley1, Best.-Nr.: 511012) von Affymetrix eingesetzt. Dieser DNA-Microarray enthält DNA-Sequenzen von 22840 Genen, die aus insgesamt 400000 EST-Sequenzen zusammengesetzt sind. Anschließend wurden die DNA-Microarrays in der Affymetrix Fluidics Station gewaschen, mit Streptavidin/ Phycoerythrin (Molecular Probes, P/N S-866) gefärbt und mit dem zugehörigen Agilent Laser Scanner (Agilent Gene Array Scanner) gescannt. Die erhaltenen Fluoreszendaten wurden mit der Software Microarray Suite 5 von Affymetrix analysiert. Nach erfolgter Qualitätskontrolle wurden alle DNA-Chip-Analysen in einer Datenbank gespeichert. Zur Ermittlung von relativen Expressionswerten für Gene (Induktions-, Repressionsfaktoren) wurden Test- und Kontroll-Chips miteinander verglichen und die in der Affymetrix-Software vorgegebenen Signifikanz-Kriterien zugrunde gelegt. Die beiden biologischen Replikate eines Experiments wurden mit je 2 Kontroll-Chips (Blindformulierung) verglichen und die erhaltenen je 4 Expressionswerte pro Gen durch Medianberechnung gemittelt. Diese Mediane sind als Induktionsfaktoren in den Ergebnistabellen angegeben. Ähnlichkeitsvergleiche von Expressionsprofilen verschiedener Experimente und Cluster-Analysen wurden mit der Software GeneMaths 1.5 von Applied Maths (Applied Maths, Keistraat 120, 9830 Sint-Martens-Latem, Belgium) durchgeführt.

Die Zusammenstellung von Gengruppen aus bestimmten Stoffwechselwegen und Signaltransduktionsketten erfolgte durch Key Word-Suche in den von Affymetrix mitgelieferten Annotationen der Gene, sowie durch Blast-Analysen (Homologievergleiche) der DNA-Target-Sequenzen, d.h. der auf dem DNA-Chip identifizierten positiven (durch Änderung des Expressionsprofils identifizierten) Sequenz, mit funktionell charakterisierten Gensequenzen aus anderen, bevorzugt pflanzlichen Organismen.

**Tabelle 1**

| Testsubstanz (Nr.): | Aufwandmenge [g a.i./ha]: | Bekannte Eigenschaft: |
|---|---|---|
| | | |
| Mefenpyr-dimethyl (1) | 150 | Safener |
| Mefenpyr-analog (2) | 150 | Safener |
| Isoxadifen-ethyl (3) | 150 | Safener |
| Cloquintocet-mexyl (4) | 150 | Safener |
| Cloquintocet-Derivat (5) | 150 | Safener |
| Pyridincarboxamid (6) | 150 | Safener |
| Salicylhydroxamat (7) | 500 | Resistenz-Induktor |
| Bion^{®}(=Acibenzolar-S-methyl) (8) | 150 | Resistenz-Induktor |
| Dichlorisonicotinsäure (9) | 150 | Resistenz-Induktor |
| Dichlorsalicylsäure (10) | 150 | Resistenz-Induktor |

Strukturformeln der verwendeten Testsubstanzen gemäß obiger Tabelle 1:

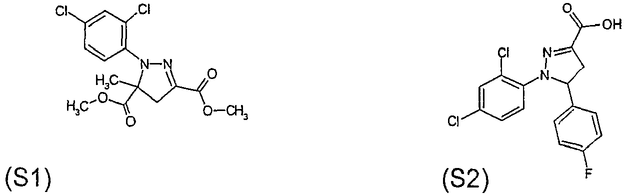

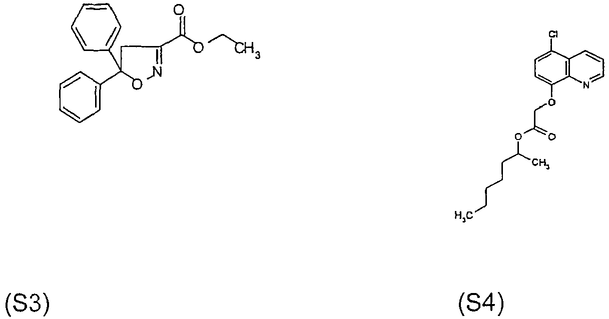

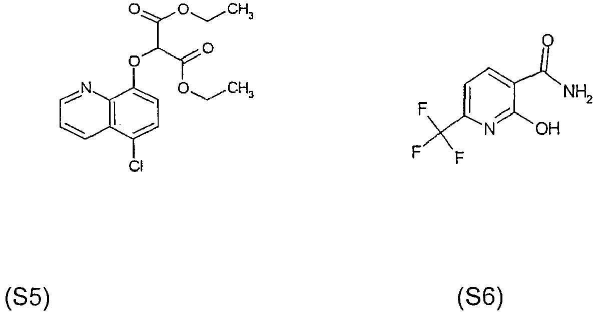

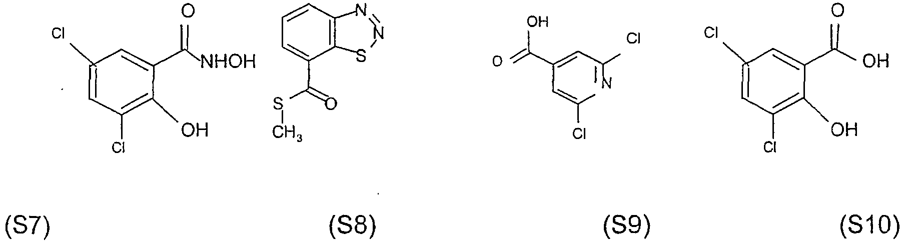

Eine Durchsicht von Gengruppen aus Signaltransduktionsketten und Stoffwechselwegen, die mit Stresstoleranz und Pathogenabwehr in Zusammenhang stehen, ergab u.a. eine starke Induktion von Genen für Protease- und Xylanase-Inhibitoren und Genen der Jasmonsäurebiosynthese (Tabelle 2a), sowie Genen für PR-Proteine und Chitinasen (Tabelle 2b), für eine Gruppe von Verbindungen, für die bisher eine Wirkung als Safener (S1-S6) bekannt ist.

**Tabelle 2a**

| "Probe Set" Nummer | S1 | S2 | S3 | S4 | S5 | S6 | S7 | S8 | S9 | S10 |
|---|---|---|---|---|---|---|---|---|---|---|
| 1.1 Contig2631_at | 1.00 | 1.00 | 97.68 1.00 | | 1.00 | 1.00 | 1.00 | 1.00 | 1.00 | 1.00 |
| 1.2 HI02E21u_s_at | 1.00 | 1.00 | 40.79 | 1.00 | 1.00 | 1.00 | 1.00 | 1.00 | 3.03 | 4.66 |
| 1.3 Contig3097 at | 1.00 | 1.00 | 8.51 | 1.00 | 1.00 | 1.00 | 1.00 | 2.11 | 1.00 | 1.00 |
| 1.4 Contig5146_at | 7.73 | 4.47 | 4.50 | 4.72 | 5.35 | 6.77 | 1.00 | 1.00 | 1.00 | 1.00 |
| 1.5 Contig 4986_at | 1.00 | 1.00 | 3.76 | 1.00 | 1.00 | 1.00 | 1.00 | 1.00 | 1.00 | 1.00 |
| 1.6 HV_Ceb0020 D05r2_s_at | 1.00 | 1.00 | 3.68 | 1.00 | 1.00 | 1.00 | 1.00 | 1.00 | 1.00 | 1.00 |
| 1.7 Contig6194_a_at | 12.91 | 4.14 | 3.25 | 2.17 | 2.81 | 3.16 | 1.00 | 1.00 | 1.00 | 1.00 |
| 1.8 Contig9556_at | 1.00 | 1.00 | 3.03 | 1.00 | 1.00 | 1.00 | 1.00 | 1.00 | 1.00 | 1.00 |
| 1.9 Contig15_a_at | 0.35 | 1.00 | 2.97 | 1.00 | 0.46 | 1.00 | 1.00 | 1.00 | 1.00 | 1.00 |
| 1.10 Contig1736_at | 1.00 | 1.00 | 2.91 | 1.00 | 1.00 | 1.00 | 1.00 | 1.00 | 1.00 | 1.00 |
| 1.11 Contig12574_at | 0.50 | 1.00 | 2.81 | 1.00 | 1.00 | 1.00 | 1.00 | 2.11 | 1.00 | 1.00 |
| 1.12 Contig6611_at | 1.00 | 1.00 | 2.41 | 1.00 | 1.00 | 1.00 | 2.31 | 3.61 | 2.00 | 2.38 |
| 1.13 Contig10030_at | 1.00 | 1.00 | 2.25 | 1.00 | 1.00 | 1.00 | 1.00 | 1.00 | 1.00 | 1.00 |
| 1.14 Contig2305_at | 1.00 | 1.00 | 2.14 | 1.00 | 1.00 | 1.00 | 41.07 | 81.57 | 12.55 | 18.13 |
| 1.15 Contig1737_at | 1.00 | 1.00 | 1.00 | 1.00 | 1.00 | 1.00 | 1.00 | 1.00 | 1.00 | 1.00 |
| 1.16 Contig3096_s_at | 1.00 | 1.00 | 1.00 | 1.00 | 1.00 | 1.00 | 2.06 | 2.68 | 1.00 | 1.00 |
| 1.17 Contig13413_at | 1.00 | 1.00 | 1.00 | 1.00 | 1.00 | 1.00 | 1.00 | 2.17 | 1.00 | 1.00 |
| 1.18 Contig2326_s_at | 2.31 | 1.00 | 1.00 | 2.45 | 1.00 | 1.00 | 1.00 | 1.00 | 1.00 | 1.00 |
| 1.19 Contig2330_x_at | 4.03 | 2.39 | 1.00 | 2.83 | 1.00 | 2.55 | 1.00 | 1.00 | 1.00 | 1.00 |
| 1.20 Contig2337_at | 4.89 | 1.00 | 1.00 | 1.00 | 1.00 | 1.00 | 1.00 | 1.00 | 1.00 | 1.00 |
| 2.1 Contig2088_s_at | 1.00 | 1.00 | 4.26 | 1.00 | 1.00 | 1.00 | 4.79 | 7.36 | 2.36 | 2.41 |
| 2.2 Contig34_s_at | 1.00 | 1.00 | 1.00 | 1.00 | 1.00 | 1.00 | 4.50 | 3.68 | 1.00 | 2.08 |
| 2.3 Contig88_x_at | 1.00 | 1.00 | 4.53 | 1.00 | 1.00 | 1.00 | 21.86 | 22.94 | 4.59 | 4.41 |
| 2.4 HD07M22r_s_at | 6.32 | 1.00 | 2.71 | 1.00 | 7.84 | 5.10 | 1.00 | 1.00 | 1.00 | 1.00 |
| 3.1 Contig8905_at | 3.10 | 2.53 | 2.99 | 2.73 | 3.01 | 2.45 | 1.00 | 1.00 | 2.53 | 1.00 |

Gemessene Expressionswerte (x-fach über dem Wert der unbehandelten Kontrolle) für:
(a) Gene der pflanzlichen Jasmonsäurebiosynthese (1.1 - 1.20)
(b) Gene, die für pflanzliche Proteinase-Inhibitoren codieren (2.1 - 2.4)
(c) Gen, das für einen pflanzlichen Xylanase-Inhibitor codiert (3.1)

Die "Probe Set"-Nummer entspricht der jeweiligen DNA-Chip-Position des Affymetrix-Chips.

Der "Probe Set"-Nummer kann mit Hilfe einer Blast-Analyse die bestmögliche korrespondierende bekannte Sequenz aus anderen annotierten Sequenzdatenbanken zugeordnet werden. Diese gemäß Blast-Analyse aufgeführten Daten sind nachfolgend aufgeführt.

"Probe Set"-Nummer korrespondierende Sequenz mit annotierter Funktion, aus allgemein zugänglicher DNA oder Proteindatenbank

| | |
|---|---|
| 1.1 | Lipase-like Protein (Oryza sativa; japonica cultivar group) |
| 1.2 | Lipoxygenase (EC 1.13.11.12) barley gbAAB70865 |
| 1.3 | Allene oxide synthase (Horedeum vulgare subsp. vulgare) |
| 1.4 | Probable 12-oxophytodienoate reductase (EC 1.3.1.42) |
| 1.5 | Allene oxide cyclase (Oryza sativa; japonica cultivar group) |
| 1.6 | Allene oxide cyclase (Oryza sativa; japonica cultivar group) |
| 1.7 | 12-oxophytodienoic acid reductase (Oryza sativa) |
| 1.8 | 12-oxophytodienoate reductase 3 (Lycopersicon esculentum) |
| 1.9 | GDSL-motif lipase/hydrolase-like protein, At5g55050.1 |
| 1.10 | Lipoxygenase 1 pir T05941 (EC 1.13.11.12) barley |
| 1.11 | Putative lipoxygenase (Oryza sativa, japonica cultivar group) |
| 1.12 | Similar to lipases (Arabidopsis thaliana; gb AAM20450.1 |
| 1.13 | Putative lipase homolog (Oryza sativa; japonica cultivar group) |
| 1.14 | Methyljasmonate inducible lipoxygenase gbAAC12951.1 |
| 1.15 | Probable lipoxygenase gbAAB60715.1 |
| 1.16 | Allen oxide synthase (Hordeum vulgare subsp. vulgare) |
| 1.17 | Similar to lipases (Arabidopsis thaliana; gb AAM20450.1) |
| 1.18 | 12-oxophytodienoic acid reductase (Oryza sativa) |
| 1.19 | 12-oxophytodienoic acid reductase (Oryza sativa) |
| 1.20 | 12-oxophytodienoate reductase (OPR1) At1g76680.1 |
| 2.1 | Bowman-Birk type trypsin inhibitor |
| 2.2 | Putative proteinase inhibitor (Hordeum vulgare subsp. vulgare) |
| 2.3 | Putative proteinase inhibitor (Hordeum vulgare subsp. vulgare) |
| 2.4 | Proteinase-Inhibitor (pir S71555, barley) |
| 3.1 | Xylanase inhibitor protein (Triticum aestivum) |

**Tabelle 2b**

| "Probe Set"- Nummer | S1 | S2 | S3 | S4 | S5 | S6 | S7 | S8 | S9 | S10 |
|---|---|---|---|---|---|---|---|---|---|---|
| 4.1 Contig2118_at | 8.69 | 1.00 | 4.86 | 1.00 | 2.33 | 2.20 | 1.00 | 1.00 | 1.00 | 1.00 |
| 4.2 Contig5369_at | 2.35 | 1.00 | 4.76 | 1.00 | 1.00 | 1.00 | 1.00 | 1.00 | 1.00 | 1.00 |
| 4.3 Contig5607_s_at | 18.64 | 15.56 | 13.36 | 18.38 | 8.75 | 9.99 | 1.00 | 1.00 | 1.00 | 1.00 |
| 5.1 Contig2990_at | 4.17 | 1.00 | 2.77 | 1.00 | 1.00 | 1.00 | 1.00 | 1.00 | 2.39 | 1.00 |
| 5.2 Contig2992_s_at | 6.50 | 1.00 | 2.19 | 1.00 | 3.12 | 2.27 | 1.00 | 1.00 | 2.14 | 1.00 |
| 5.3 Contig5023_at | 9.51 | 3.18 | 1.00 | 1.00 | 2.73 | 1.00 | 1.00 | 1.00 | 4.56 | 1.00 |

Gemessene Expressionswerte (x-fach über dem Wert der unbehandelten Kontrolle) für
(a) Gene, die für pflanzliche PR-Proteine codieren (4.1 - 4.3)
(b) Gene, die für pflanzliche Chitinasen codieren (5.1 - 5.3)

Die "Probe Set"-Nummer entspricht der jeweiligen DNA-Chip-Position des Affymetrix-Chips.

Der "Probe Set"-Nummer kann mit Hilfe einer Blast-Analyse die bestmögliche korrespondierende bekannte Sequenz aus anderen annotierten Seqeunzdatenbanken zugeordnet werden. Diese gemäß Blast-Analyse aufgeführten Daten sind nachfolgend aufgeführt.

"Probe Set"- Nummer korrespondierende Sequenz mit annotierter Funktion, aus allgemein zugänglicher DNA oder Proteindatenbank

| | |
|---|---|
| 4.1 | Peroxidase (EC 1.11.1.7), pathogen-induced (barley) |
| 4.2 | Pathogen related protein; (Oryza sativa; gbAAL74406.1) |
| 4.3 | Pathogen-related protein (barely; PirT06168) |
| 5.1 | Chitinase (EC 3.2.1.14) (Barley; embCAA55344.1) |
| 5.2 | Chitinase (EC 3.2.1.14) (Barley; embCAA55344.1) |
| 5.3 | Class III chitinase (Oryza sativa subsp. japonica; gbAAM08773.1) |

Die aus diesen Tabellen abgeleiteten Induktionsmuster, die direkt durch die erhaltenen Expressionswerte dargestellt werden, zeigen charakteristische Unterschiede zwischen Safenern und Resistenzinduktoren, wobei die Wirkung auf die Jasmonsäure-Biosynthese beim Isoxadifen am stärksten ausgeprägt ist.

Die gefundenen Expressionsmuster erlauben das Auffinden von Substanzen mit einer ähnlichen Signatur und legen den Schluß auf eine gleichartige Wirkung dieser Substanzen in der Aktivierung der pflanzlichen Pathogenabwehr nahe.

### Beispiel 2

### Abstoßende Wirkung von Safener-behandelten Pflanzen auf phytopathogene Insekten:

Gerstepflanzen (Sorte Baronesse) wurden wie in Beispiel 1 beschrieben angezogen und nach 10 Tagen mit 150 g a.i./ha Isoxadifen-ethyl (S 3) bzw. Blindformulierung durch Spritzapplikation behandelt. Die Versuche wurden in Replikaten von je 10 Töpfen durchgeführt.

Alle Töpfe wurden 6 h bzw. 24 h nach Substanzapplikation mit einer Population der phytopathogenen Blattlaus *Rhopalosiphum padi* gleichmäßig beimpft. Die Versuche wurden nach 7 Tagen und 14 Tagen durch Auszählen der an den Blättern befindlichen Tiere ausgewertet.

Die Blattlauspopulationen waren auf den Safener-behandelten Pflanzen gegenüber den Kontrollen nach 7 Tagen um durchschnittlich 50 % und nach 14 Tagen um durchschnittlich 70 % reduziert.

Eine direkte Toxizität von Isoxadifen-ethyl (S3) gegen Blattläuse konnte im Sachez-Test ohne Pflanzen nicht nachgewiesen werden.

### Beispiel 3

### Nachweis von erhöhtem Oxophytodienoat (OPDA) - und Jasmonat (JA)-Konzentrationen in Isoxadifen-behandelten Pflanzen

Gerstepflanzen (Varietät Baronesse) wurden gemäß den in in Beispiel 1 beschriebenen Konditionen angezogen und durch Spritzapplikation mit Safener Isoxadifen-ethyl (S 3) behandelt.

Die Aufwandmengen waren wie folgt gewählt:
(1) 30 [g a.i./ha] ; (2) 150 [g a.i./ha] ; (3) Blindformulierung (kein Wirkstoff)

Die Pflanzenblätter wurden nach der Behandlung zu verschiedenen Zeitpunkten (h=Stunden) geerntet, und zwar nach 1 h, 2h, 4h, 6h, 12h, 24h und 48h. Alle Proben waren jeweils als Replikate von 3 Töpfen angelegt.

Die Aufarbeitung der Octadecanoide und Jasmonate aus den Blättern wurde nach einer in der Literatur beschriebenen Methode durchgeführt (Müller A, Düchting P und Weiler EW (2002), A multiplex GC-MS / MS technique for the sensitive and quantitative single-run analysis of acidic phytohormones and related compounds, and its application to Arabidopsis thaliana. Planta, 216, 44-56).

Für jeden Messpunkt wurden 300mg Pflanzenmaterial extrahiert.

Zur internen Standardisierung wurden 50 pmol [¹³C]2-JA und 100 pmol [17,17,17,18,18-²H]-cis-OPDA eingesetzt. Die Aufreinigung der Extrakte erfolgte über Aminopropyl-Anionenaustausch-Chromatographie in selbst hergestellten Miniatur-Festphasenaustauscher-Säulen.

Die Ergebnisse sind in den Tabellen 3a bis 3d dargestellt, wobei angegebenen Werte jeweils Mittelwerte aus den 3 Replikaten darstellen.

**Tabelle 3a**

| Gemessene Oxophytodienoat (OPDA)-Konzentration in pmol/g Blattgewebe | | |
|---|---|---|
| **Zeit / Stunden** | **Kontrolle** | **Isoxadifen-ethyl (S3) 30 g a.i/ha** |
| 0 | 2500 | 2500 |
| 1 | 1500 | 6500 |
| 2 | 1000 | 3750 |
| 4 | 2500 | 7000 |
| 6 | 2500 | 9000 |

**Tabelle 3 b**

| Gemessene Oxophytodienoat (OPDA)-Konzentration in pmol/g Blattgewebe | | |
|---|---|---|
| **Zeit / Stunden** | **Kontrolle** | **Isoxadifen-ethyl (S3) 150 g a.i./ha** |
| 0 | 2600 | 2600 |
| 1 | 1600 | 2800 |
| 2 | 1200 | 1800 |
| 4 | 2600 | 2200 |
| 6 | 2700 | 5100 |
| 12 | 3600 | 2400 |
| 24 | 2800 | 5100 |
| 48 | 1800 | 1900 |

**Tabelle 3 c**

| Gemessene Jasmonat (JA)-Konzentration in pmol/g Blattgewebe | | |
|---|---|---|
| **Zeit / Stunden** | **Kontrolle** | **Isoxadifen-ethyl (S3) 30 g a.i/ha** |
| 0 | 140 | 140 |
| 1 | 180 | 600 |
| 2 | 70 | 320 |
| 4 | 190 | 790 |
| 6 | 130 | 620 |

**Tabelle 3 d**

| Gemessene Jasmonat (JA)-Konzentration in pmol/g Blattgewebe | | |
|---|---|---|
| **Zeit / Stunden** | **Kontrolle** | **Isoxadifen-ethyl (S3) 150 g a.i./ha** |
| 0 | 140 | 140 |
| 1 | 175 | 290 |
| 2 | 70 | 280 |
| 4 | 190 | 270 |
| 6 | 135 | 275 |
| 12 | 220 | 155 |
| 24 | 145 | 265 |
| 48 | 135 | 320 |

Die Konzentrationen an Oxophytodienoat (OPDA)_lagen im Bereich von 1800-9000 pmol/g Blattgewebe, bei Jasmonat (JA) im Bereich von 70-800 pmol/g Blattgewebe. Nach Behandlung mit Isoxadifen-ethyl (S 3) konnte ein deutlicher Anstieg der Konzentrationen von beiden Substanzen bis auf das ca. 5 fache der Werte in den Blindformulierungsproben ohne Wirkstoff gemessen werden.

## Patentansprüche

1. Verwendung von Isoxadifen-ethyl zum Schutz von Nutzpflanzen in Nutzpflanzenkulturen gegenüber phytopathogenen Insekten.

2. Verwendung gemäß Anspruch 1, wobei Isoxadifen-ethyl in Nutzpflanzenkulturen in Kombination mit direkt gegen phytopathogene Insekten wirksame Verbindungen eingesetzt wird, und wobei die Applikation entweder gleichzeitig oder zeitlich versetzt erfolgt.

3. Verwendung gemäß Anspruch 2, wobei es sich bei den direkt gegen phytopathogene Insekten wirksame Verbindungen um Verbindungen aus der Gruppe der Ketoenole oder um Agonisten bzw. Antagonisten des nicotinergen Acetylcholinrezeptors handelt.

4. Verwendung gemäß Anspruch 3, wobei die Agonisten bzw. Antagonisten des nicotinergen Acetylcholinrezeptors ausgewählt sind aus der Gruppe der Nitromethylene und Nitroimine.

## Claims

1. Use of isoxadifen-ethyl for protecting useful plants in crops of useful plants against phytopathogenic insects.

2. Use according to Claim 1, wherein isoxadifen-ethyl is employed in crops of useful plants in combination with compounds with direct activity against phytopathogenic insects, and wherein the application is carried out either simultaneously or staggered in time.

3. Use according to Claim 2, wherein the compounds with direct activity against phytopathogenic insects are compounds from the group of the ketoenols or agonists or antagonists of the nicotinergic acetylcholin receptor.

4. Use according to Claim 3, wherein the agonists or antagonists of the nicotinergic acetylcholin receptor are selected from the group of the nitromethylenes and nitroimines.

## Revendications

1. Utilisation d'isoxadifène-éthyle pour la protection de plantes utiles, dans des cultures de plantes utiles, contre des insectes phytopathogènes.

2. Utilisation selon la revendication 1, dans laquelle on utilise l'isoxadifène-éthyle, dans des cultures de plantes utiles, en association avec des composés agissant directement contre des insectes phytopathogènes, et dans laquelle l'application s'effectue soit simultanément, soit en étant décalée dans le temps.

3. Utilisation selon la revendication 2, dans laquelle les composés agissant directement contre des insectes phytopathogènes consistent en des composés choisis dans le groupe des cétoénols ou en des agonistes ou antagonistes du récepteur nicotinergique d'acétylcholine.

4. Utilisation selon la revendication 3, dans laquelle les agonistes ou antagonistes du récepteur nicotinergique d'acétylcholine sont choisis dans le groupe des nitrométhylènes et nitro-imines.
